# EUROPEAN PATENT SPECIFICATION

(11) **EP 2 490 292 B1**
(45) Date of publication and mention of the grant of the patent: **04.05.2016**
(21) Application number: 12150404.7
(22) Date of filing: 06.01.2012
(51) Int. Cl.: H01M 10/0525, H01M 10/0569, H01M 10/42

(54) **Electrolyte for a rechargeable lithium battery, and rechargeable lithium battery including the same**
Elektrolyt für eine wiederaufladbare Lithiumbatterie und wiederaufladbare Lithiumbatterie denselben umfassend
Électrolyte pour batterie rechargeable au lithium, et batterie rechargeable au lithium le comprenant

(30) Priority: 16.02.2011 KR 20110013769
(43) Date of publication of application: 22.08.2012
(73) Proprietor: SAMSUNG SDI CO., LTD., Yongin-si, Gyeonggi-do (KR)
(72) Inventor: Park, Dai-In, Gyeonggi-do (KR); Yang, Ho-Seok, Gyeonggi-do (KR); Han, Man-Seok, Gyeonggi-do (KR); Lim, Jin-Hyunk, Gyeonggi-do (KR); Oh, Mi-Hyeun, Gyeonggi-do (KR)
(74) Representative: Mounteney, Simon James

(56) References cited:
- EP-A1- 1 508 934
- WO-A1-01/29920
- JP-A- 2010 141 276
- KR-A- 20080 043 034
- US-A1- 2004 013 946
- US-A1- 2011 207 000

## Description

### BACKGROUND

### 1. Field

This disclosure relates to an electrolyte for a rechargeable lithium battery, and a rechargeable lithium battery including the same.

### 2. Description of the Related Art

Lithium rechargeable batteries have recently drawn attention as a power source for small portable electronic devices. They use an organic electrolyte and thereby have two or more times the discharge voltage of a comparable battery using an alkali aqueous solution, and accordingly have high energy density.

This rechargeable lithium battery is formed by injecting an electrolyte into a battery cell including a positive electrode including a positive active material that can intercalate and deintercalate lithium, and a negative electrode including a negative active material that can intercalate and deintercalate lithium.

When the rechargeable lithium battery is overcharged, safety of the battery is not secured and cycle-life (life cycle) of the battery is deteriorated.

KR 2008 0043034 A discloses an electrolyte for secondary battery including (a) an electrolytic salt, (b) an electrolytic solvent, (c) a first fluorine-containing compound that is oxidized above the operating voltage of a positive electrode to generate heat, and (d) a second compound, such as 4-methylbenzonitrile, which is oxidized in the voltage range higher than oxidation potential of the first compound to generate gas.

Documents JP 2010 141276 A, EP 1 508 934 A1 & US 2004/013946 A1 all describe electrolyte compositions comprising a non-aqueous organic solvent and a nitrile compound, such as benzonitrile or a halogenated derivative thereof, as additive or co-solvent.

WO 01/29920 A1 discloses the use of methoxy-substituted benzonitrile compounds as additives in electrolyte solutions for secondary batteries.

### SUMMARY

An aspect of an embodiment of the present invention is directed toward a electrolyte for a rechargeable lithium battery that not only provides for superb safety when the rechargeable lithium battery is overcharged, but also provides for an excellent cycle-life (life cycle).

An aspect of an embodiment of the present invention is directed toward a rechargeable lithium battery including the electrolyte.

According to an embodiment of the present invention, an electrolyte for a rechargeable lithium battery is provided to include a lithium salt, a succinonitrile additive, a non-aqueous organic solvent; and an alkyl benzonitrile compound represented by the following Chemical Formula 1.

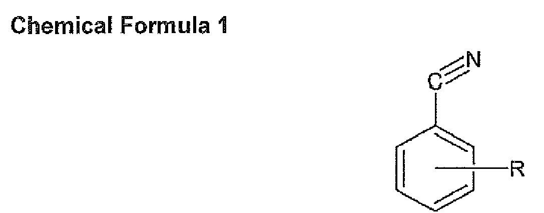

In Chemical Formula 1,
R is a substituted or unsubstituted C1 to C5 alkyl group.

The alkyl benzonitrile compound preferably may be a methyl benzonitrile compound represented by the following Chemical Formula 2.

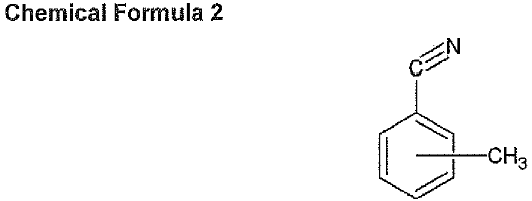

The alkyl benzonitrile compound preferably may be a 4-alkyl benzonitrile compound represented by the following Chemical Formula 3.

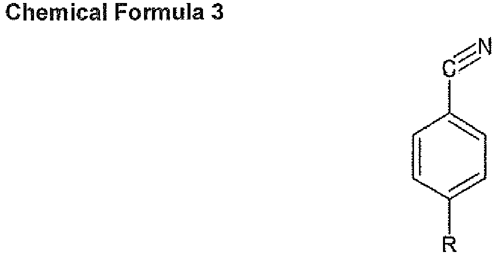

In Chemical Formula 3,
R is a substituted or unsubstituted C1 to C5 alkyl group.

The alkyl benzonitrile compound preferably may be a 4-methyl benzonitrile compound represented by the following Chemical Formula 4.

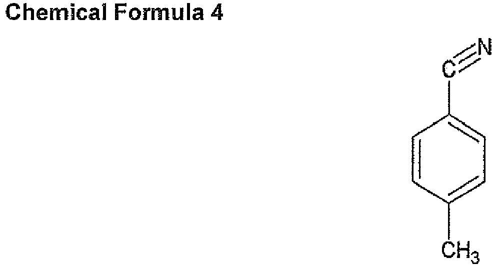

The alkyl benzonitrile compound preferably may be included in an amount of more than 0 wt% and less than or equal to 25 wt% based on the total amount of the solvent of the present invention.

The alkyl benzonitrile compound may have a peak between 4.90 V and 5.50 V when linear sweep voltammetry (LSV) is measured.

According to another embodiment of the present invention, a rechargeable lithium battery is provided to include a positive electrode; a negative electrode; and an electrolyte including a lithium salt, a non-aqueous organic solvent and the alkyl benzonitrile compound represented by the above Chemical Formula 1.

Other embodiments of the present invention are described below in more detail.

When the electrolyte according to an embodiment of the present invention is used in a rechargeable lithium battery, the rechargeable lithium battery may have not only superb safety during an overcharge, but also excellent cycle-life.

### BRIEF DESCRIPTION OF THE DRAWINGS

FIG. 1 is a schematic view of a rechargeable lithium battery according to one embodiment of the present invention.
FIG, 2 is a linear sweep voltammetry (LSV) graph of electrolytes according to Example 2 and Comparative Example 1.
FIG. 3 is a graph enlarging the LSV graph of FIG. 2.
FiG. 4 is a graph showing the state of the rechargeable lithium battery cell according to Example 2 when the rechargeable lithium batteries are overcharged.

### DETAILED DESCRIPTION

Embodiments of the present invention will hereinafter be described in more detail.

As used herein, when a specific definition is not otherwise provided, the term "substituted" refers to one substituted with one selected from either a halogen (e.g. a fluorine, chlorine, bromine or iodine, preferably fluorine), a hydroxyl group, a C1 to C20 alkyl group (preferably a C1 to C10 alkyl group), a C2 to C20 alkenyl group (preferably a C2 to C10 alkenyl group), a C2 to C20 alkynyl group (preferably a C2 to C10 alkynyl group), a C1 to C20 alkoxy group (preferably a C1 to C10 alkoxy group), a C3 to C30 cycloalkyl group (preferably a C3 to C10 cycloalkyl group), a C3 to C30 cycloalkenyl group (preferably a C4 to C10 cycloalkenyl group), a C7 to C30 cycloalkynyl group (preferably a C7 to C10 cycloalkynyl group), a C2 to C30 heterocycloalkyl group (preferably a C2 to C10 heterocycloalkyl group, a C2 to C30 heterocycloalkenyl group (preferably a C2 to C10 heterocycloalkenyl group), a C2 to C30 heterocycloalkynyl group (preferably a C2 to C10 heterocycloalkynyl group), a C6 to C30 aryl group (preferably a C6 to C10 aryl group), a C6 to C30 aryloxy group (preferably a C6 to C10 aryloxy group), a C2 to C30 heteroaryl group (preferably a C2 to C10 heteroaryl group), an amine group (-NR'R", wherein R' and R" are the same or different and are either hydrogen, a C1 to C20 alkyl group (preferably a C1 to C10 alkyl group), or a C6 to C30 aryl group(preferably a C6 to C10 aryl group), an ester group (-COOR"', wherein R'" is a C1 to C20 alkyl group (preferably a C1 to C10 alkyl group), or a C6 to C30 aryl group (preferably a C6 to C10 aryl group), a carboxyl group (-COOH), a nitro group (-NO₂ or a cyano group (-CN), instead of at least one hydrogen.

As used herein, when a specification definition is not otherwise provided, the prefix "hetero" refers to a functional group including 1 to 5 heteroatoms, preferably 1 to 3 heteroatoms selected from the group of N, O, P, and S or a combination thereof.

The electrolyte for a rechargeable lithium battery according to the invention includes a lithium salt, a succinonitrile additive, a non-aqueous organic solvent, and an alkyl benzonitrile compound.

In one embodiment, the alkyl benzonitrile compound is represented by the following Chemical Formula 1.

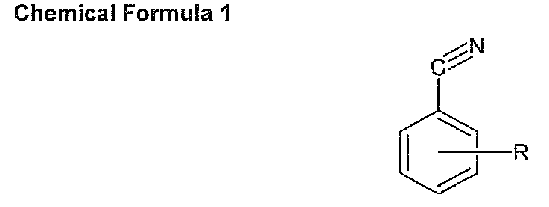

In Chemical Formula 1, R is a substituted or unsubstituted C1 to C5 alkyl.

Examples of the alkyl benzonitrile compound include a substituted or unsubstituted methyl benzonitrile compound, an ethyl benzonitrile compound, a propyl benzonitrile compound, a butyl benzonitrile compound, or a pentyl benzonitrile compound.

Among them, the methyl benzonitrile compound represented by the following Chemical Formula 2 is used in one embodiment.

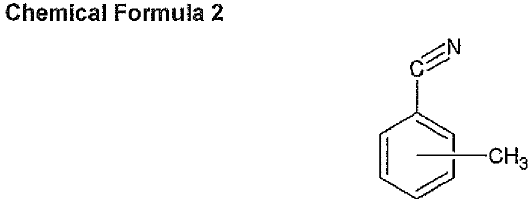

Also, an alkyl group in the alkyl benzonitrile compound may exist in all hydrogen positions bonded to the carbons of a benzene ring, and the examples of such alkyl benzonitrile compound include a 2-alkyl benzonitrile compound, a 3-alkyl benzonitrile compound, and a 4-alkyl benzonitrile compound.

Among them, the 4-alkyl benzonitrile compound represented by the following Chemical Formula 3 is used in one embodiment.

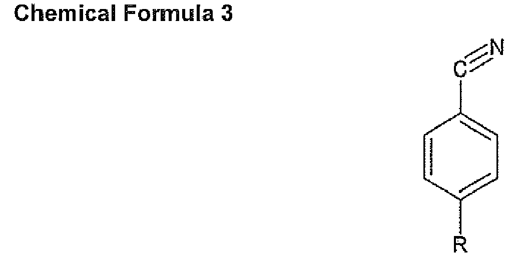

In Chemical Formula 3, R is a substituted or unsubstituted C1 to C5 alkyl.

Among the alkyl benzonitrile compound, the 4-methyl benzonitrile compound represented by the following Chemical Formula 4 is used in one embodiment.

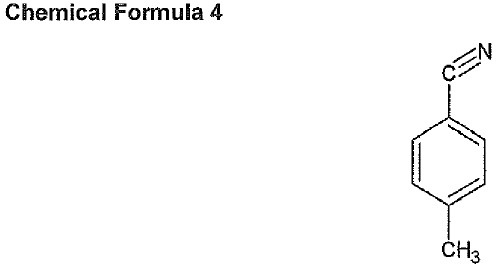

The alkyl benzonitrile compound preferably may be included in an amount of more than about 0 wt% and less than or equal to about 25 wt% based on the total amount of the solvent of the present invention, i.e. the total weight of the combination of alkyl benzonitrile, non-aqueous organic solvent and any further components that may be present, preferably more than about 0 wt% and less than or equal to about 20 wt%, more preferably more than about 0 wt% and less than or equal to about 15 wt%. According to one embodiment, the alkyl benzonitrile compound is preferably included in an amount of about 0.5 wt% to about 10 wt%, and according to another embodiment, the alkyl benzonitrile compound is included in an amount of about 1 wt% to about 7 wt%. For example, the alkyl benzonitrile compound is preferably included in an amount of about 0.5 wt% to about 20 wt%, more preferably an amount of about 1 wt% to about 17 wt%, more preferably an amount of about 3 wt% to about 15 wt%.

The alkyl benzonitrile compound may have a peak between about 4.90 V and about 5.50 V during the measurement of linear sweep voltammetry (LSV), and according to one embodiment, a peak is between about 5.00 V and about 5.15 V. When the alkyl benzonitrile compound having R (i.e. an alkyl group) at 2-position or 4-position, the alkyl benzonitile compound preferably has a peak between about 4.90V and about 5.30V. When an electrolyte includes the alkyl benzonitrile compound, decomposition of the alkyl benzonitrile is started at a voltage lower than the decomposition starting voltage of the non-aqueous organic solvent. As a result, the LSV measurement result shows that the additive is decomposed at a voltage lower than the decomposition starting voltage of the non-aqueous organic solvent during an overcharge, and heat generated from the decomposition cuts off a protection device to thereby prevent or protect from an overcharge from occurring.

The LSV is measured at a scanning speed of about 0.05 mV/s to about 1.0 mV/s in a voltage range of about 3 V to about 7V. Preferably, the scanning rate is about 0.1 mV/s. In one embodiment, when the LSV is measured, a platinum electrode is used as a working electrode, and lithium metal is used as a reference electrode and a counter electrode.

The lithium salt supplies lithium ions in the battery, provides a basic operation of the rechargeable lithium battery, and improves lithium ion transport between positive and negative electrodes.

Examples of the lithium salt include at least one supporting salt selected from the group consisting of LiPF₆, LiBF₄, LiSbF₆, LiAsF₆, LiN(SO₃C₂F₅)₂, LiC₄F₉SO₃, LiClO₄, LiAlO₂, LiAlCl₄, LiN(CₓF₂ₓ₊₁SO₂)(C_{y}F₂y₊₁SO₂) - where x and y are natural numbers, LiCl, Lil, LiB(C₂O₄)₂ (lithium bis(oxalato) borate: LiBOB), and combinations thereof.

The concentration of the lithium salt may preferably range from about 0.1 M to about 2.0M. In one embodiment, when the lithium salt is included at the above concentration range, electrolyte performance and lithium ion mobility is enhanced due to desired electrolyte conductivity and viscosity.

The non-aqueous organic solvent acts as a medium for transmitting ions taking part in the electrochemical reaction of the battery. The non-aqueous organic solvent may include a carbonate-based compound, an ester-based compound, an ether-based compound, a ketone-based compound, an alcohol-based compound, an aprotic solvent, or a combination thereof. In a preferred embodiment the non-aqueous organic solvent contains a carbonate-based compound, preferably a mixture of carbonate-based compounds.

The non-aqueous organic solvent is preferably included in an amount of 75 wt% to 99.9 wt%, more preferably an amount of 80 wt% to 99.5 wt%, more (preferably an amount of 85 wt% to 95 wt% based on the total weight of the solvent of the present invention.

The carbonate-based compound may include a linear carbonate compound, a cyclic carbonate compound, or a combination thereof.

The linear carbonate compound may include diethyl carbonate (DEC), ethylmethyl carbonate (EMC), dimethyl carbonate (DMC), dipropyl carbonate (DPC), methylpropyl carbonate (MPC), ethylpropyl carbonate (EPC), or a combination thereof; and the cyclic carbonate compound may include ethylene carbonate (EC), propylene carbonate (PC), butylene carbonate (BC), fluoroethylene carbonate (FEC), vinylethylene carbonate (VEC), or a combination thereof. Preferably, the carbonate compound is EC, EMC, DEC, FEC or VEC or a combination thereof, optionally a combination of at least two of these, preferably at least three of these and more preferably a combination of all of these five compounds.

The linear carbonate compound preferably may be added at about 60 wt% or more based on the total amount of the non-aqueous organic solvent, preferably about 60 wt% to about 100 wt%, more preferably about 75 wt% to about 90 wt%, more preferably about 80 wt% to about 85 wt%. The cyclic carbonate compound preferably may be added at about 40 wt% or less based on the total amount of the non-aqueous organic solvent, preferably about 0 wt% to about 40 wt%, more preferably about 10 wt% to about 25 wt%, more preferably about 15 wt% to about 20 wt%,. In one embodiment, when the linear carbonate compound and the cyclic carbonate compound are respectively included within the range, it provides the solvent with both a high dielectric constant and a low viscosity.

The ester-based compound may include methylacetate, ethyl acetate, n-propylacetate, 1,1-dimethylethylacetate, methylpropionate, ethylpropionate, γ-butyrolactone, decanolide, valerolactone, mevalonolactone, caprolactone, or the like. The ether-based compound may include dibutylether, tetraglyme, diglyme, dimethoxyethane, 2-methyltetrahydrofuran, tetrahydrofuran, or the like. The ketone-based compound may include cyclohexanone or the like. The alcohol-based compound may include ethanol, isopropyl alcohol, or the like.

The aprotic solvent may include nitriles such as R-CN (wherein R is a C₂ to C₂₀ linear, branched, or cyclic hydrocarbon, a double bond, an aromatic ring, or an ether bond); amides such as dimethylformamide; dioxolanes such as 1,3-dioxolane, sulfolanes; or the like.

The non-aqueous organic solvent preferably may be used alone or as a mixture. When the organic solvent is used as a mixture, the mixing ratio may be controlled in accordance with desirable battery performance.

The electrolyte according to the invention includes succinonitrile as an additive. The succinonitrile preferably may be included in an amount of 0.1 wt% to 5 wt% based on the total amount of the electrolyte. According to one embodiment, the succinonitrile preferably may be included in an amount of 0.5 wt% to 3 wt%. When the electrolyte includes the succinonitrile, the succinonitrile forms a coordinate covalent bond with the transition element of a positive active material at the positive electrode interface to thereby suppress open circuit voltage (OCV) defects during a formation process, and improve thermal stability at a high temperature such as heat exposure.

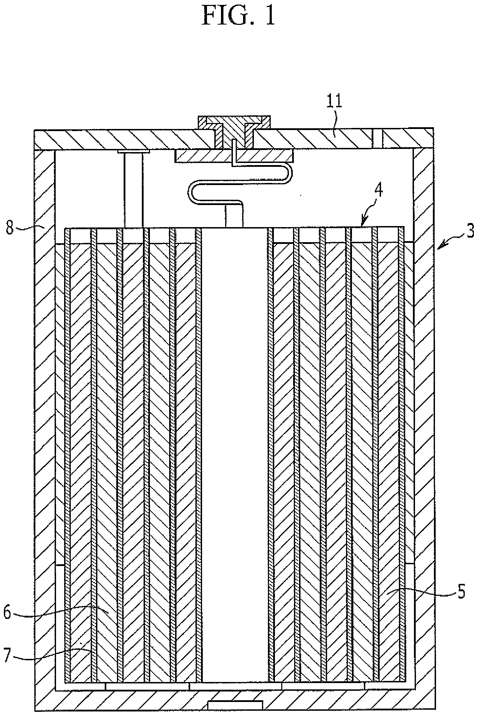

Hereafter, a rechargeable lithium battery including the electrolyte is described with reference to FIG. 1.

FIG. 1 is a schematic view of a representative structure of a rechargeable lithium battery according to one embodiment.

Referring to FIG. 1, the rechargeable lithium battery 3 is a prismatic battery that includes an electrode assembly 4 in a battery case 8, an electrolyte implanted through the upper portion of the case 8, and a cap plate 11 sealing the case 8. The electrode assembly 4 includes a positive electrode 5, a negative electrode 6, and a separator 7 positioned between the positive electrode 5 and the negative electrode 6. The rechargeable lithium battery of the present invention is not limited to a prismatic form of rechargeable lithium battery, and it may be formed in diverse forms such as a cylindrical form, a coin-type form, or a pouch form as long as it includes the electrolyte for the rechargeable lithium battery and operates as the battery.

The electrolyte is the same as described according to embodiments of the present invention.

The positive electrode 5 includes a current collector and a positive active material layer disposed on the current collector. The positive active material layer includes a positive active material, a binder, and a conductive material.

The current collector may include aluminum (Al), but is not limited thereto.

The positive active material includes lithiated intercalation compounds that reversibly intercalate and deintercalate lithium ions. The positive active material may include a composite oxide including cobalt, manganese, and/or nickel, as well as lithium. In one embodiment, the following lithium-containing compounds preferably may be used, but is not limited thereto:

LiₐA_{1-b}B_{b}D₂(0.90 ≤ a ≤ 1.8 and 0 ≤ b ≤ 0.5); LiₐE_{1-b}B_{b}O_{2-c}D_{c}(0.90 ≤ a ≤ 1.8, 0 ≤ b ≤ 0.5, 0 ≤ c ≤ 0.05); LiE_{2-b}B_{b}O_{4-c}D_{c}(0 ≤ b ≤ 0.5, 0 ≤ c ≤ 0.05); LiₐNi_{1-b-c}Co_{b}B_{c}D_{α}(0.90 ≤ a ≤ 1.8, 0 ≤ b ≤ 0.5, 0 ≤ c ≤ 0.05, 0 < α ≤ 2); LiₐNi_{1-b-c}Co_{b}B_{c}O_{2-α}F_{α}(0.90 ≤ a ≤ 1.8, 0 ≤ b ≤ 0.5, 0 ≤ c ≤ 0.05, 0 < α < 2); LiₐNi_{1-b-c}Co_{b}B_{c}O_{2-α}F₂(0.90 ≤ a ≤ 1.8, 0 ≤ b ≤ 0.5, 0 ≤ c ≤ 0.05, 0 < α < 2); LiₐNi_{1-b-c}Mn_{b}B_{c}D_{α}(0.90 ≤ a ≤ 1.8, 0 ≤ b ≤ 0.5, 0 ≤ c ≤ 0.05, 0 < α ≤ 2); LiₐNi_{1-b-c}M_{nb}B_{c}O_{2-α}F_{α}(0.90 ≤ a ≤ 1.8, 0 ≤ b ≤ 0.5, 0 ≤ c ≤ 0.05, 0 < α < 2); LiₐNi_{1-b-c}Mn_{b}B_{c}O_{2-α}F₂ (0.90 ≤ a ≤ 1.8, 0 ≤ b ≤ 0.5, 0 ≤ c ≤ 0.05, 0 < α < 2); LiₐNi_{b}E_{c}G_{d}O₂ (0.90 ≤ a ≤ 1.8, 0 ≤ b ≤ 0.9, 0 ≤ c ≤ 0.5, 0.001 ≤ d ≤ 0.1.); LiₐNi_{b}Co_{c}Mn_{d}GeO₂ (0.90 ≤ a ≤ 1.8, 0 ≤ b ≤ 0.9, 0 ≤ c ≤ 0.5,0 ≤ d ≤ 0.5,0.001 ≤ e ≤ 0.1); LiₐNiG_{b}O₂ (0.90 ≤ a ≤ 1.8, 0.001 ≤ b ≤ 0.1.); LiₐCoGₑO₂(0.90 a ≤ 1.8, 0.001 ≤ b:5 0.1.); LiₐMnG_{b}O₂(0.90 a ≤ 1.8, 0.001 ≤ b ≤ 0.1.); LiₐMn₂G_{b}O₄ (0.90 ≤ a ≤ 1.8, 0.001 ≤ b ≤ 0.1.); QO₂; QS₂; LiQS₂; V₂O₅; LiV₂O₅; LiIO₂; LiNiVO₄; Li_{(3-f)}J₂(PO₄)₃ (0 ≤ f ≤ 2); Li_{(3-f)}Fe₂(PO₄)₃ (0 ≤ f ≤ 2); and/or LiFePO₄.

In the above chemical formula, A is Ni, Co, Mn, or a combination thereof; R is Al, Ni, Co, Mn, Cr, Fe, Mg, Sr, V, a rare earth element, or a combination thereof; D is O, F, S, P, or a combination thereof; E is Co, Mn, or a combination thereof; Z is F, S, P, or a combination thereof; G is Al, Cr, Mn, Fe, Mg, La, Ce, Sr, V, or a combination thereof; Q is Ti, Mo, Mn, or a combination thereof; T is Cr, V, Fe, Sc, Y, or a combination thereof; and J is V, Cr, Mn, Co, Ni, Cu, or a combination thereof.

The positive active material may include the positive active material with the coating layer, or a compound of the active material and the active material coated with the coating layer. The coating layer may include at least one coating element compound selected from the group consisting of an oxide of the coating element, a hydroxide of the coating element, an oxyhydroxide of the coating element, an oxycarbonate of the coating element, and a hydroxycarbonate of the coating element. The compound for the coating layer preferably may be either amorphous or crystalline. The coating element or coating material included in the coating layer preferably may be selected from the group consisting of Mg, Al, Co, K, Na, Ca, Si, Ti, V, Sn, Ge, Ga, B, As, Zr, or a mixture thereof. The coating process may include any suitable process as long as it does not cause any side effects on the properties of the positive active material (e.g., spray coating, immersing), which is well known to persons having ordinary skill in this art, so a detailed description thereof is omitted.

The binder improves binding properties of the positive active material particles to each other and to a current collector. Examples of the binder include polyvinyl alcohol, carboxylmethyl cellulose, hydroxypropyl cellulose, diacetyl cellulose, polyvinyl chloride, carboxylated polyvinyl chloride, polyvinyl fluoride, polymer including ethylene oxide, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and the like, but are not limited thereto.

The conductive material improves electrical conductivity of a negative electrode. Any electrically conductive material can be used as a conductive agent unless it causes a chemical change. Non-limiting examples of the conductive material include natural graphite, artificial graphite, carbon black, acetylene black, ketjen black, a carbon fiber, a metal powder or a metal fiber of copper, nickel, aluminum, silver, and a polyphenylene derivative, which preferably may be used singularly or as a mixture thereof.

The negative electrode 6 includes a current collector and a negative active material layer disposed thereon.

The current collector may include a copper foil, a nickel foil, a stainless steel foil, a titanium foil, a nickel foam, a copper foam, a polymer substrate coated with a conductive metal, or a combination thereof, but is not limited thereto.

The negative active material layer may include a negative active material, a binder, and optionally, a conductive material.

The negative active material includes a material that reversibly intercalates/deintercalates lithium ions, a lithium metal, a lithium metal alloy, a material being capable of doping/dedoping lithium, and/or a transition metal oxide.

The material that can reversibly intercalate/deintercalate lithium ions includes a carbon material. The carbon material may be any suitable carbon-based negative active material in a lithium ion rechargeable battery. Examples of the carbon material include crystalline carbon, amorphous carbon, and mixtures thereof. The crystalline carbon preferably may be non-shaped, or sheet, flake, spherical, or fiber shaped natural graphite or artificial graphite. The amorphous carbon preferably may be a soft carbon, a hard carbon, a mesophase pitch carbonization product, fired coke, or the like.

Examples of the lithium metal alloy include lithium and a metal selected from the group consisting of Na, K, Rb, Cs, Fr, Be, Mg, Ca, Sr, Si, Sb, Pb, In, Zn, Ba, Ra, Ge, Al, Sn, and combinations thereof.

Examples of the material being capable of doping lithium include Si, SiOₓ (0 < x < 2), an Si-Y alloy (where Y is an element selected from the group consisting of an alkali metal, an alkali-earth metal, a group 13 element, a group 14 element, a group 15 element, a group 16 element, a transition element, a rare earth element, and combinations thereof, and is not Si), Sn, SnO₂ an Sn-Y alloy (where Y is an element selected from the group consisting of an alkali metal, an alkali-earth metal, a group 13 element, a group 14 element, a group 15 element, a group 16 element, a transition element, a rare earth element, and combinations thereof, and is not Sn), and mixtures thereof. At least one of these materials preferably may be mixed with SiO₂. The element Y preferably may be selected from the group consisting of Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Ge, P, As, Sb, Bi, S, Se, Te, Po, and combinations thereof.

Examples of the transition metal oxide include vanadium oxide, lithium vanadium oxide, and the like.

The binder improves binding properties of negative active material particles with one another and with a current collector. Examples of the binder include polyvinyl alcohol, carboxylmethyl cellulose, hydroxypropyl cellulose, polyvinyl chloride, carboxylated polyvinyl chloride, polyvinyl fluoride, polymer including ethylene oxide, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and the like, but are not limited thereto.

The conductive material is included to improve electrode conductivity. Any electrically conductive material may be used as a conductive material unless it causes a chemical change. Examples of the conductive material include carbon-based materials such as natural graphite, artificial graphite, carbon black, acetylene black, ketjen black, carbon fibers, and the like; metal-based materials of metal powder or metal fiber including copper, nickel, aluminum, silver; conductive polymers such as polyphenylene derivatives; and mixtures thereof.

The positive electrode 5 and the negative electrode 6 may be fabricated by a method including mixing an active material and a binder, optionally a conductive material, and a binder into an active material composition and coating the composition on a current collector.

The electrode manufacturing method is well known, and thus is not described in detail in the present specification. The solvent preferably may be N-methylpyrrolidone, but it is not limited thereto.

The separator 7 preferably may be formed as a single layer or a multilayer, and preferably may be made of polyethylene, polypropylene, polyvinylidene fluoride, or a combination thereof.

Hereinafter, the embodiments are illustrated in more detail with reference to examples. However, the following are exemplary embodiments and are not limiting.

Furthermore, what is not described in this specification can be sufficiently understood by those who have knowledge in this field and will not be illustrated here.

### Preparation of Electrolyte Solution

### Examples 1 to 24 and Comparative Examples 1 to 10

1.0M LiPF₆ was dissolved in a mixed solvent of ethylene carbonate (EC), ethylmethyl carbonate (EMC) and diethyl carbonate (DEC) at a volume ratio of about 3:5:2, to prepare an electrolyte precursor. Electrolyte solutions were prepared by adding 5 wt% of fluoroethylene carbonate (FEC), 1 wt% of vinylethylene carbonate (VEC), 0.2 wt% of LiBF₄ 1 wt% of succinonitrile and alkyl benzonitrile compounds according to the kinds and contents shown in the following Table 1, to the balance amount of the electrolyte precursor.

**Table 1**

| | Alkyl benzonitrile compound | Amount of alkyl Benzonitrile compound (wt%) |
|---|---|---|
| Example 1 | 4-methyl benzonitrile | 0.5 |
| Example 2 | 4-methyl benzonitrile | 3 |
| Example 3 | 4-methyl benzonitrile | 5 |
| Example 4 | 4-methyl benzonitrile | 7 |
| Example 5 | 4-methyl benzonitrile | 10 |
| Example 6 | 4-methyl benzonitrile | 15 |
| Example 7 | 3-methyl benzonitrile | 0.5 |
| Example 8 | 3-methyl benzonitrile | 5 |
| Example 9 | 3-methyl benzonitrile | 15 |
| Example 10 | 2-methyl benzonitrile | 0.5 |
| Example 11 | 2-methyl benzonitrile | 5 |
| Example 12 | 2-methyl benzonitrile | 15 |
| Example 13 | 4-ethyl benzonitrile | 10 |
| Example 14 | 3-ethyl benzonitrile | 15 |
| Example 15 | 2-ethyl benzonitrile | 15 |
| Example 16 | 4-propyl benzonitrile | 10 |
| Example 17 | 3-propyl benzonitrile | 15 |
| Example 18 | 2-propyl benzonitrile | 15 |
| Example 19 | 4-butyl benzonitrile | 10 |
| Example 20 | 3-butyl benzonitrile | 15 |
| Example 21 | 2-butyl benzonitrile | 15 |
| Example 22 | 4-pentyl benzonitrile | 10 |
| Example 23 | 3-pentyl benzonitrile | 15 |
| Example 24 | 2-pentyl benzonitrile | 15 |
| Comparative Example 1 | - | - |
| Comparative Example 2 | biphenyl | 0.5 |
| Comparative Example 3 | biphenyl | 5 |
| Comparative Example 4 | biphenyl | 15 |
| Comparative Example 5 | cyclohexyl benzene | 0.5 |
| Comparative Example 6 | cyclohexyl benzene | 5 |
| Comparative Example 7 | cyclohexyl benzene | 15 |
| Comparative Example 8 | benzonitrile | 0.5 |
| Comparative Example 9 | benzonitrile | 5 |
| Comparative Example 10 | benzonitrile | 15 |

### Fabrication of Rechargeable Lithium Battery Cells

A composition for a positive active material layer was prepared by mixing LiCoO₂ as a positive active material, polyvinylidene fluoride (PVDF) as a binder, and carbon as a conductive material at a weight ratio of about 92:4:4, and dispersing the mixture in N-methyl-2-pyrrolidone. A positive electrode was fabricated by coating a 20 µm-thick aluminum foil with the composition for a positive active material layer, drying it, and compressing it.

A composition for a negative active material layer was prepared by mixing a crystalline artificial graphite as a negative active material and polyvinylidene fluoride (PVDF) as a binder in a weight ratio of about 92:8, and dispersing the mixture in N-methyl-2-pyrrolidone.

A negative electrode was fabricated by coating a 15 µm-thick copper foil with the composition for a negative active material layer, drying it, and compressing it.

Rechargeable lithium battery cells were fabricated by winding and compressing the positive and negative electrodes (that were fabricated through the above description) with a separator which was of a 25 µm-thick polyethylene material, and inserting the resultant structure into a prismatic (1200mA) case of about 30mm×48mm×6mm. Herein, the electrolyte solutions prepared according to Examples 1 to 24 and Comparative Examples 1 to 10 were used.

### Experimental Example 1: Measurement of Decomposition Initiation Voltage of Electrolyte Solution

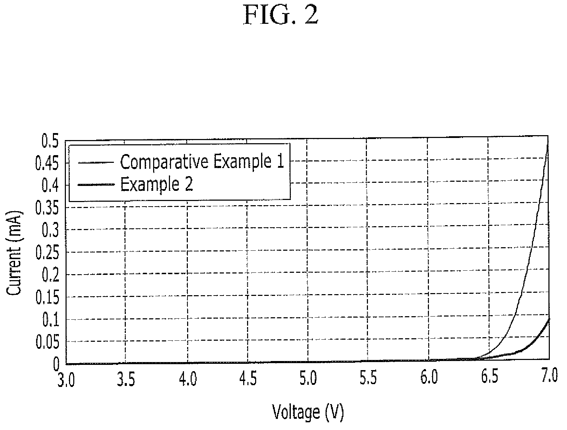

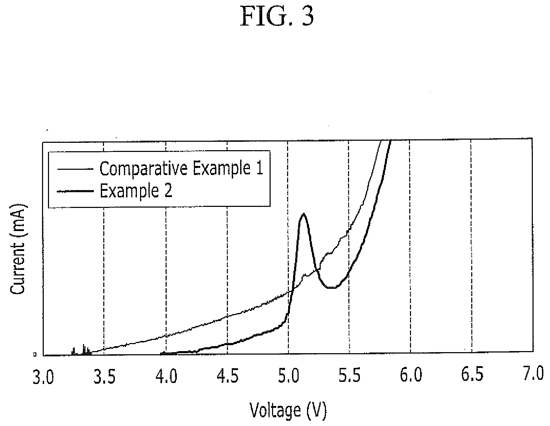

The decomposition initiation voltages of the electrolyte solutions prepared according to Example 2 and Comparative Example 1 were measured using a linear sweep voltammetry (LSV), and the measurement results were presented in FIGS. 2 and 3.

Measurement of LSV was performed within a voltage range of about 3 V to about 7 V at a scanning rate of about 0.1 mV/s, a platinum electrode was used as a working electrode, and lithium metal was used as a reference electrode and a counter electrode. The LSV measurement instrument used a Multi-Channel Potentiostat (produced by Wona Tech) WMPG1000.

FIG. 2 is an LSV graph of the electrolyte solutions prepared according to Example 2 and Comparative Example 1, and FIG. 3 is a graph enlarging the LSV graph FIG. 2.

Referring to FIGS. 2 and 3, it may be seen that in the case of Example 2, the alkyl benzonitrile compound begins to be decomposed at a voltage lower than the decomposition initiation voltage of a non-aqueous organic solvent. Also, in the case of the electrolyte solution prepared according to Example 2, an oxidation peak appeared at about 5.1 V, and the decomposition initiation voltage of the electrolyte solution prepared according to Example 2 was higher than the decomposition initiation voltage of that of Comparative Example 1. Accordingly, it may be seen from the LSV measurement result that excellent safety may be secured because the decomposition of the additive was initiated at a voltage lower than the decomposition initiation voltage of a non-aqueous organic solvent during an overcharge, and the heat generated from the decomposition of the additive initiated the operation of (i.e., cut off) a protection device to thereby prevent or protect from an overcharge.

### Experimental Example 2: Evaluation of State of Rechargeable Lithium Battery Cell During Overcharge

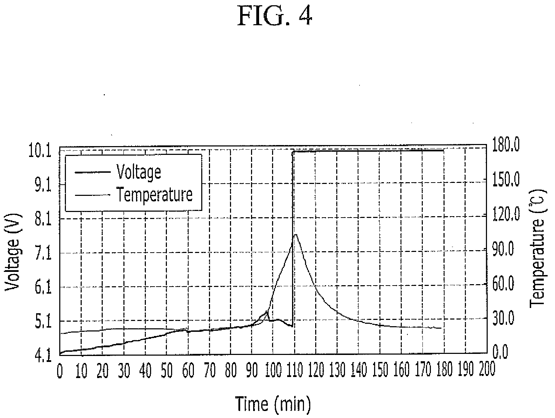

The rechargeable lithium battery cell prepared according to Example 2 was overcharged, and the state according to voltage and temperature was measured. The measurement results were presented in FIG. 4.

The rechargeable lithium battery cell prepared according to Example 2 were cut-off charged in the conditions of about 1200mA, 4.2V, and 33 min, and charged with constant current and constant voltage of about 950mA and 10V.

FIG. 4 is a graph showing overcharge states of the rechargeable lithium battery cell prepared according to Example 2. It can be seen from FIG. 4 that the temperature was increased to about 100°C and then decreased, and the voltage was increased to about 10V and then maintained. The increase in temperature was brought by decomposing the additive in the electrolyte solution during the overcharging to generate heat and the decreases in temperature was by removing the additive caused from the decomposition. The increases in voltage were brought about by cutting off the protection device caused from the generated heat. This may be described as a mechanism in which an alkyl benzonitrile compound is decomposed prior to a non-aqueous organic solvent during an overcharge.

### Experimental Example 3: Pack Overcharge Test of Rechargeable Lithium Battery Cell

Packs the rechargeable lithium battery cells prepared according to Examples 1 to 24 and Comparative Examples 1 to 10 i) were cut-off charged in the cut-off conditions of about 1220mA, 4.2V, and 33 minutes under a standard environment and then the charging was paused for about 10 minutes to about 72 hours or less, and ii) a secondary protection device (which is a thermal fuse: EYP2MP098DUK) was attached to each of the rechargeable lithium battery cells (evaluated as a single cell). iii) The pack was placed on a wood plate, and charged with constant current and constant voltage of about 950mA and about 10V for about 5 hours. iv) After the maximal temperature evaluation, the safety during an overcharge was evaluated by observing OCV, the phenomenon occurred during an overcharge, and the measurement result was presented in the following Table 2.

### Experimental Example 4: Hot Plate Test of Rechargeable Lithium Battery Cells

The safeties of the rechargeable lithium battery cells prepared according to Examples 1 to 24 and Comparative Examples 1 to 10 during an overcharge were evaluated by i) performing a cut-off charge in the conditions of about 950mA, 4.2V, and 0.05C (60mA) under a standard environment and pausing the charging for about 10 minutes to about 72 hours or less, ii) placing the rechargeable lithium battery cells on a hot plate set to about 250°C, and iii) determining the phenomenon that occurred during an overcharge. The results were presented in the following Table 2.

The safety evaluation standards that were measured during an overcharge in Experimental Examples 1 and 2 were as follows.

The number before L denotes the number of test cells (e.g., 6L, 4L, 10L, etc.), and L0: Excellent, L1: liquid leakage, L2: flash, L3: smoke, L4: ignition, L5: breakage.

### Experimental Example 5: Evaluation of Cycle-Life of Rechargeable Lithium Battery Cells

The rechargeable lithium battery cells prepared according to Examples 1 to 24 and Comparative Examples 1 to 10 were charged at about 1C to the charge voltage of about 4.2V under the condition of constant current-constant voltage (CC-CV) and discharged at about 1C to the cut-off voltage of about 3.0V under the CC condition. The charge and discharge were performed 300 times and the capacity retention based on cycle was measured to evaluate the cycle-life, and the evaluation result was presented in the following Table 2.

**Table 2**

| | Alkyl benzonitrile compound | Amount of alkyl benzonitrile compound (wt%) | Pack overcharge test | Hot plate test | Capacity retention (%) (300^{th} capacity relative to first capacity) |
|---|---|---|---|---|---|
| Example 1 | 4-methyl benzonitrile | 0.5 | 6L0,4L1 | 6L0,4L1 | 97 |
| Example 2 | 4-methyl benzonitrile | 3 | 10L0 | 10L0 | 96 |
| Example 3 | 4-methyl benzonitrile | 5 | 10L0 | 10L0 | 95 |
| Example 4 | 4-methyl benzonitrile | 7 | 10L0 | 10L0 | 94 |
| Example 5 | 4-methyl benzonitrile | 10 | 10L0 | 10L0 | 93 |
| Example 6 | 4-methyl benzonitrile | 15 | 10L0 | 10L0 | 91 |
| Example 7 | 3-methyl benzonitrile | 0.5 | 5L0,5L1 | 5L0,5L1 | 96 |
| Example 8 | 3-methyl benzonitrile | 5 | 9L0,1L1 | 9L0,1L1 | 89 |
| Example 9 | 3-methyl benzonitrile | 15 | 10L0 | 10L0 | 87 |
| Example 10 | 2-methyl benzonitrile | 0.5 | 5L0,5L1 | 5L0,5L1 | 97 |
| Example 11 | 2-methyl benzonitrile | 5 | 10L0 | 10L0 | 90 |
| Example 12 | 2-methyl benzonitrile | 15 | 10L0 | 10L0 | 88 |
| Example 13 | 4-ethyl benzonitrile | 10 | 10L0 | 10L0 | 90 |
| Example 14 | 3-ethyl benzonitrile | 15 | 10L0 | 10L0 | 83 |
| Example 15 | 2-ethyl benzonitrile | 15 | 10L0 | 10L0 | 85 |
| Example 16 | 4-propyl benzonitrile | 10 | 10L0 | 10L0 | 88 |
| Example 17 | 3-propyl benzonitrile | 15 | 10L0 | 10L0 | 81 |
| Example 18 | 2-propyl benzonitrile | 15 | 10L0 | 10L0 | 82 |
| Example 19 | 4-butyl benzonitrile | 10 | 10L0 | 10L0 | 86 |
| Example 20 | 3-butyl benzonitrile | 15 | 10L0 | 10L0 | 79 |
| Example 21 | 2-butyl benzonitrile | 15 | 10L0 | 10L0 | 80 |
| Example 22 | 4-pentyl benzonitrile | 10 | 10L0 | 10L0 | 83 |
| Example 23 | 3-pentyl benzonitrile | 15 | 10L0 | 10L0 | 76 |
| Example 24 | 2-pentyl benzonitrile | 15 | 10L0 | 10L0 | 77 |
| Comparative Example 1 | - | - | 10L5 | 10L5 | 98 |
| Comparative Example 2 | biphenyl | 0.5 | 10L5 | 10L5 | 97 |
| Comparative Example 3 | biphenyl | 5 | 5L0,5L5 | 4L0,6L5 | 80 |
| Comparative Example 4 | biphenyl | 15 | 8L0,2L5 | 5L0,5L5 | 70 |
| Comparative Example 5 | cyclohexyl benzene | 0.5 | 10L5 | 10L5 | 98 |
| Comparative Example 6 | cyclohexyl benzene | 5 | 4L0,6L5 | 3L0,7L5 | 80 |
| Comparative Example 7 | cyclohexyl benzene | 15 | 7L0,3L5 | 5L0,5L5 | 72 |
| Comparative Example 8 | benzonitrile | 0.5 | 2L0,8L5 | 2L0,8L5 | 93 |
| Comparative Example 9 | benzonitrile | 5 | 3L0,7L5 | 4L0,6L5 | 90 |
| Comparative Example 10 | benzonitrile | 15 | 5L0,5L5 | 5L0,5L5 | 85 |

It may be seen from Table 2 that those of Examples 1 to 24 using the alkyl benzonitrile compound prepared according to an embodiment of the present invention as a component for an electrolyte solution, had both excellent safety during an overcharge and excellent cycle-life, compared with those of Comparative Examples 1 to 10.

Also, it may be seen from Examples 1 to 24 that when an alkyl group existed in the same position, the safety during an overcharge and the cycle-life were better when methyl benzonitrile compound was used, than when ethyl benzonitrile compound, propyl benzonitrile compound, butyl benzonitrile compound, and pentyl benzonitrile compound were used.

Also, it may be seen from Examples 1 to 24 that in the case of an alkyl group of the same kind, the safety during an overcharge and cycle-life were better when the 4-alkyl benzonitrile compound was used, than when the 3-alkyl benzonitrile compound and the 2-alkyl benzonitrile compound were used.

Also, those of Examples 1 to 6 using a 4-methyl benzonitrile compound showed most improvement in safety during an overcharge while still have high cycle-life.

## Claims

1. An electrolyte for a rechargeable lithium battery, the electrolyte consisting of:
a lithium salt;
a succinonitrile additive;
a non-aqueous organic solvent; and
an alkyl benzonitrile compound represented by the following Chemical Formula 1: 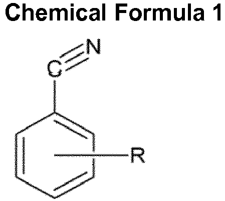 wherein,
R is a substituted or unsubstituted C1 to C5 alkyl group.

2. The electrolyte of claim 1, wherein the alkyl benzonitrile compound comprises a methyl benzonitrile compound represented by the following Chemical Formula 2:

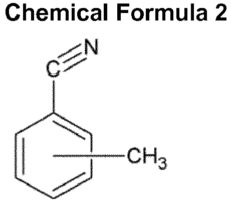

3. The electrolyte of claim 1, wherein the alkyl benzonitrile compound comprises a 4-alkyl benzonitrile compound represented by the following Chemical Formula 3: 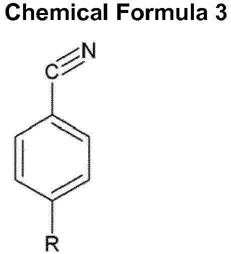 wherein,
R is a substituted or unsubstituted C1 to C5 alkyl group.

4. The electrolyte of claim 1, wherein the alkyl benzonitrile compound comprises a 4-methyl benzonitrile compound represented by the following Chemical Formula 4.

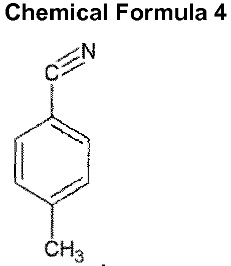

5. The electrolyte of any of claims 1 to 4, wherein the alkyl benzonitrile compound is included in an amount of more than 0 wt% and less than or equal to 25 wt% based on the total amount of the solvent solution.

6. The electrolyte of any of claims 1 to 5, wherein the alkyl benzonitrile compound shows a peak between 4.90V and 5.50 V when linear sweep voltammetry (LSV) is measured.

7. The electrolyte of any of claims 1 to 5, wherein the non-aqueous organic solvent comprises a carbonate-based compound, an ester-based compound, an ether-based compound, a ketone-based compound, an alcohol-based compound, an aprotic solvent, or a combination thereof.

8. The electrolyte of claim 7, wherein the organic solvent contains a carbonate-based compound, preferably a mixture of carbonate-based compounds, preferably a mixture of a linear carbonate compound and a cyclic carbonate compound.

9. The electrolyte of claim 8, wherein the solvent is a combination of ethylene carbonate (EC), ethylmethyl carbonate (EMC), diethyl carbonate (DEC), fluoroethylene carbonate (FEC) and vinylethylene carbonate (VEC).

10. The electrolyte of any of claims 1 to 9, wherein the lithium salt includes at least one supporting salt selected from the group consisting of LiPF₆, LiBF₄, LiSbF₆, LiAsF₆, LiN(SO₃C₂F₅)₂, LiC₄F₉SO₃, LiClO₄, LiAlO₂, LiAlCl₄, LiN(CₓF₂ₓ₊₁SO₂)(C_{y}F_{2y+1}SO₂) - where x and y are natural numbers, LiCl, Lil, LiB(C₂O₄)₂ (lithium bis(oxalato) borate: LiBOB), and combinations thereof.

11. The electrolyte of any of claims 1 to 10, wherein the succinonitrile additive is included in an amount of 0.1 wt% to 5 wt% based on the total amount of the electrolyte.

12. The electrolyte of any of claims 1 to 11, wherein the non-aqueous organic solvent is present in an amount of 75 wt% to 99.9 wt% based on the total weight of the solvent.

13. A rechargeable lithium battery comprising:
a positive electrode;
a negative electrode; and
an electrolyte according to any of claims 1 to 12.

## Patentansprüche

1. Elektrolyt für eine wiederaufladbare Lithiumbatterie, wobei der Elektrolyt besteht aus:
einem Lithiumsalz;
einem Succinonitril-Zusatz;
einem nichtwässrigen organischen Lösungsmittel; und
einer Alkylbenzonitrilverbindung der folgenden chemischen Formel 1: 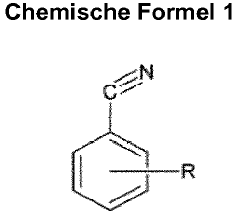 wobei
R ein substituierter oder unsubstituierter C₁- bis C₅-Alkylrest ist.

2. Elektrolyt nach Anspruch 1, wobei die Alkylbenzonitrilverbindung eine Methylbenzonitrilverbindung der folgenden chemischen Formel 2 umfasst:

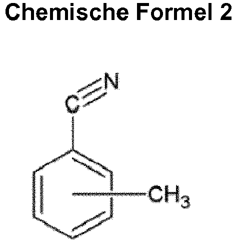

3. Elektrolyt nach Anspruch 1, wobei die Alkylbenzonitrilverbindung eine 4-Alkylbenzonitrilverbindung der folgenden chemischen Formel 3 umfasst: 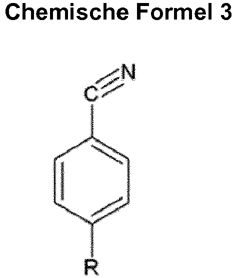 wobei
R ein substituierter oder unsubstituierter C₁- bis C₅-Alkylrest ist.

4. Elektrolyt nach Anspruch 1, wobei die Alkylbenzonitrilverbindung eine 4-Methylbenzonitrilverbindung der folgenden chemischen Formel 4 umfasst:

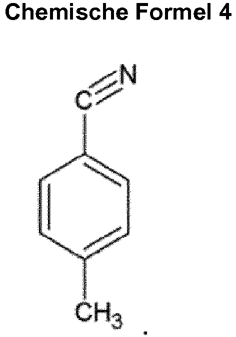

5. Elektrolyt nach einem der Ansprüche 1 bis 4, wobei die Alkylbenzonitrilverbindung in einer Menge von mehr als 0 Gew.-% und weniger als oder gleich 25 Gew.-%, bezogen auf die Gesamtmenge der Lösungsmittellösung, enthalten ist.

6. Elektrolyt nach einem der Ansprüche 1 bis 5, wobei die Alkylbenzonitrilverbindung bei Durchführung von Linear-Sweep-Voltammetrie (LSV) einen Peak zwischen 4,90 V und 5,50 V zeigt.

7. Elektrolyt nach einem der Ansprüche 1 bis 5, wobei das nichtwässrige organische Lösungsmittel eine Verbindung auf Carbonatbasis, eine Verbindung auf Esterbasis, eine Verbindung auf Etherbasis, eine Verbindung auf Ketonbasis, eine Verbindung auf Alkoholbasis, ein aprotisches Lösungsmittel oder eine Kombination davon umfasst.

8. Elektrolyt nach Anspruch 7, wobei das organische Lösungsmittel eine Verbindung auf Carbonatbasis, vorzugsweise ein Gemisch aus Verbindungen auf Carbonatbasis, vorzugsweise ein Gemisch aus einer linearen Carbonatverbindung und einer cyclischen Carbonatverbindung, enthält.

9. Elektrolyt nach Anspruch 8, wobei das Lösungsmittel eine Kombination aus Ethylencarbonat (EC), Ethylmethylcarbonat (EMC), Diethylcarbonat (DEC), Fluorethylencarbonat (FEC) und Vinylethylencarbonat (VEC) ist.

10. Elektrolyt nach einem der Ansprüche 1 bis 9, wobei das Lithiumsalz mindestens ein Leitsalz, ausgewählt aus der Gruppe bestehend aus LiPF₆, LiBF₄, LiSbF₆, LiAsF₆, LiN(SO₃C₂F₅)₂, LiC₄F₉SO₃, LiClO₄, LiAlO₂, LiAlCl₄, LiN(CₓF₂ₓ₊₁SO₂)(C_{y}F_{2y+1}SO₂), - wobei x und y natürliche Zahlen sind, LiCl, Lil, LiB(C₂O₄)₂ (Lithiumbis(oxalato)borat: LiBOB), und Kombinationen davon, umfasst.

11. Elektrolyt nach einem der Ansprüche 1 bis 10, wobei der Succinonitril-Zusatz in einer Menge von 0,1 Gew.-% bis 5 Gew.-%, bezogen auf die Gesamtmenge des Elektrolyten, enthalten ist.

12. Elektrolyt nach einem der Ansprüche 1 bis 11, wobei das nichtwässrige organische Lösungsmittel in einer Menge von 75 Gew.-% bis 99,9 Gew.-%, bezogen auf das Gesamtgewicht des Lösungsmittels, enthalten ist.

13. Wiederaufladbare Lithiumbatterie, umfassend:
eine positive Elektrode;
eine negative Elektrode; und
einen Elektrolyt nach einem der Ansprüche 1 bis 12.

## Revendications

1. Electrolyte pour batterie rechargeable au lithium, l'électrolyte consistant en :
un sel de lithium ;
un additif du type succinonitrile ;
une solvant organique non aqueux ; et
un composé du type alkyl-benzonitrile représenté par la formule chimique 1 suivante : 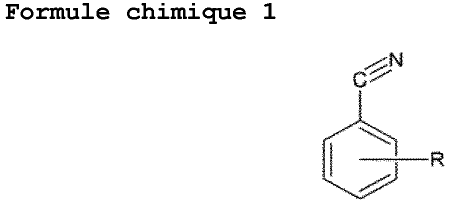 dans laquelle :
R représente un groupe alkyle en C₁ à C₅ substitué ou non substitué.

2. Electrolyte suivant la revendication 1, dans lequel le composé du type alkyl-benzonitrile consiste en un composé du type méthyl-benzonitrile représenté par la formule chimique 2 suivante :

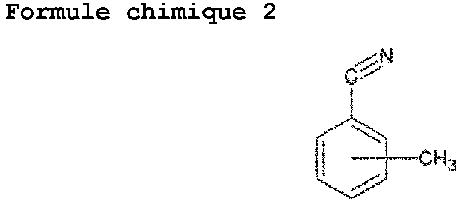

3. Electrolyte suivant la revendication 1, dans lequel le composé du type alkyl-benzonitrile consiste en un composé du type 4-alkyl-benzonitrile représenté par la formule chimique 3 suivante : 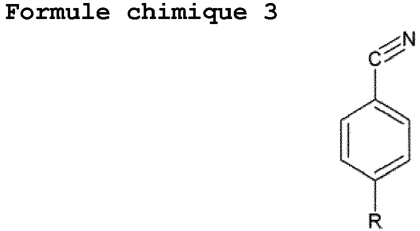 dans laquelle :
R représente un groupe alkyle en C₁ à C₅ substitué ou non substitué.

4. Electrolyte suivant la revendication 1, dans lequel le composé du type alkyl-benzonitrile consiste en un composé du type 4-méthyl-benzonitrile représenté par la formule chimique 4 suivante :

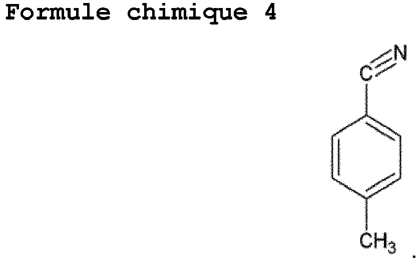

5. Electrolyte suivant l'une quelconque des revendications 1 à 4, dans lequel le composé du type alkyl-benzonitrile est inclus en une quantité supérieure à 0 % en poids et inférieure ou égale à 25 % en poids sur la base totale de la solution de solvant.

6. Electrolyte suivant l'une quelconque des revendications 1 à 5, dans lequel le composé du type alkyl-benzonitrile présente un pic entre 4,90 V et 5,50 V lors de la mesure par voltammétrie à balayage linéaire (LSV).

7. Electrolyte suivant l'une quelconque des revendications 1 à 5, dans lequel le solvant organique non aqueux comprend un composé à base de carbonate, un composé à base d'ester, un composé à base d'éther, un composé à base de cétone, un composé à base d'alcool, un solvant aprotique ou une de leurs associations.

8. Electrolyte suivant la revendication 7, dans lequel le solvant organique contient un composé à base de carbonate, de préférence un mélange de composés à base de carbonate, de préférence un mélange d'un composé du type carbonate linéaire et d'un composé du type carbonate cyclique.

9. Electrolyte suivant la revendication 8, dans lequel le solvant est une association de carbonate d'éthylène (EC), de carbonate d'éthylméthyle (EMC), de carbonate de diéthyle (DEC), de carbonate de fluoréthylène (FEC) et de carbonate de vinyléthylène (VEC).

10. Electrolyte suivant l'une quelconque des revendications 1 à 9, dans lequel le sel de lithium comprend au moins sel de support choisi dans le groupe consistant en LiPF₆, LiBF₄, LiSbF₆, LiAsF₆, LiN(SO₃C₂F₅)₂, LiC₄F₉SO₃, LiClO₄, LiAlO₂, LiAlCl₄, LiN(CₓF₂ₓ₊₁SO₂)(C_{y}F_{2y+1}SO₂) - où x et y sont des nombres entiers, LiCl, LiI, LiB(C₂O₄)₂ (bis (oxalato) borate de lithium : LiBOB), et leurs associations.

11. Electrolyte suivant l'une quelconque des revendications 1 à 10, dans lequel l'additif du type succinonitrile est inclus en une quantité de 0,1 % en poids à 5 % en poids sur la base de la quantité totale de l'électrolyte.

12. Electrolyte suivant l'une quelconque des revendications 1 à 11, dans lequel le solvant organique non aqueux est présent en une quantité de 75 % en poids à 99,9 % en poids sur la base du poids total du solvant.

13. Batterie au lithium rechargeable, comprenant :
une électrode positive ;
une électrode négative ; et
un électrolyte suivant l'une quelconque des revendications 1 à 12.
